# EUROPEAN PATENT SPECIFICATION

(11) **EP 1 074 244 B1**
(45) Date of publication and mention of the grant of the patent: **08.03.2006**
(21) Application number: 00116499.5
(22) Date of filing: 31.07.2000
(51) Int. Cl.: A61K 8/67, A61K 8/895, A61Q 17/04

(54) **Topical composition comprising a tocopherol compound**
Eine Tocopherol-Verbindung enthaltende topische Zusammensetzung
Composition topique comprenant un composé tocophérol

(30) Priority: 06.08.1999 EP 99115555
(43) Date of publication of application: 07.02.2001
(73) Proprietor: DSM IP Assets B.V., 6411 TE Heerlen (NL)
(72) Inventor: Huber, Ulrich, 8703 Erlenbach (CH); Schwarzenbach, Rolf, 8405 Winterthur (CH)
(74) Representative: Schwander, Kuno

(56) References cited:
- WO-A-93/10745
- DE-A- 19 735 900

## Description

The present invention relates to topical compositions useful to prevent damage to skin caused by ultraviolet irradiation, such as sunburn and sun-induced premature aging of the skin.

In particular the invention relates to topical compositions comprising a tocopherol compound and a lipophilic polysiloxane type UV-B screening agent.

Topical compositions containing tocopherol (Vitamin E) and UV screening agents are known in the art. US Patent No. 4,144,325 describes for example a sunscreen composition comprising a tocopherol compound and an ultraviolet-absorbing compound selected from the group consisting of salicylates, para-aminobenzoates, benzophenone, cinnamate, naphthoate, gallate and mixtures thereof.

Topical compositions containing tocopherol sorbate as active agent, together with sunscreening agents are described in US Patent No. 4,847,072. The sunscreening agent is selected from the group consisting of 2-ethylhexyl p-methoxycinnamate, butylmethoxy dibenzoylmethane, 2-hydroxy-4-methoxybenzopenone, octyl dimethyl p-aminobenzoic acid, the 4-N,N-(2-ethylhexyl) methylaminobenzoic acid ester of 2,4-dihydroxy benzophenone, the N,N-di-(2-ethylhexyl)-4-aminobenzoic acid ester of 4-hydroxy dibenzoylmethane, the 4-N,N-(2-ethylhexyl)methylaminobenzoic acid ester of 4-hydroxydibenzoylmethane, the 4-N,N-(2-ethylhexyl)methylaminobenzoic acid ester of 2-hydroxy-4-(2-hydroxyethoxy)benzophenone, the 4-N,N-(2-ethylhexyl)methylaminobenzoic acid ester of 4-(2-hydroxy-ethoxy) dibenzylmethane, the N-N-di-(2-ethylhexyl)-4-aminobenzoic acid ester of 2-hydroxy-4-(2-hydroxyethoxy)benzophenone, the N,N-di(2-ethylhexyl)-4-aminobenzoic acid ester of 4-(2-hydroxyethoxy)dibenzoylmethane and mixtures thereof. The pharmaceutical/cosmetic compositions disclosed therein may also include a penetration enhancing agent to enhance penetration of tocopherol sorbate into the skin. The penetration enhancing agent is e.g. N-(2-hydroxyethyl)pyrrolidone.

It has now been found that the penetration rate of tocopherol compounds can be enhanced by adding a lipophilic polysiloxane type UV-B screening agent without a further penetration enhancing agent.

Thus, the present invention is concerned with the use of a linear or cyclic polysiloxane compound of the general formula Ia or Ib, 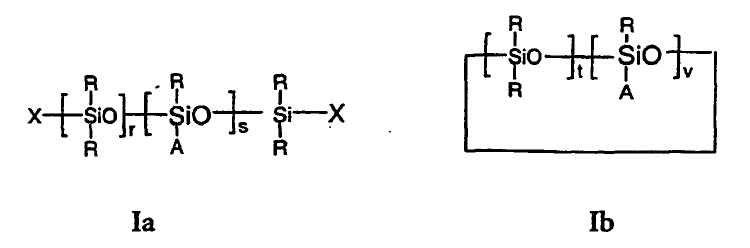 wherein
- X: signifies R or A;
- A: signifies a group of the general formula IIa, IIb or IIc; 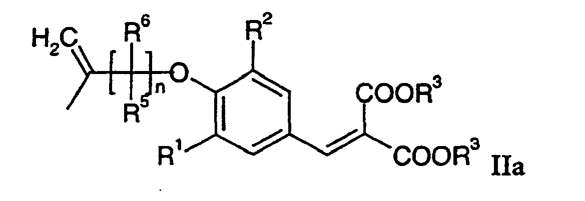 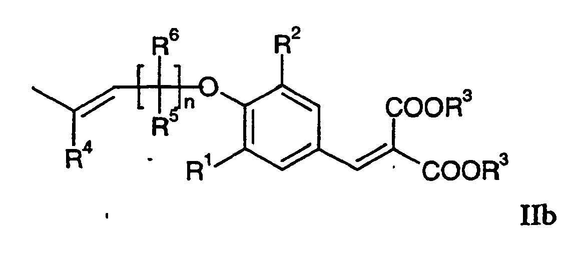 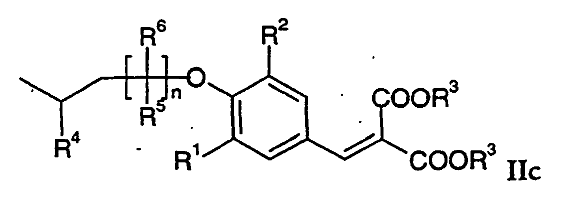
- R: signifies hydrogen, C₁₋₆ alkyl or phenyl;
- R¹ and R²: each independently signify hydrogen, hydroxy, C₁₋₆-alkyl or C₁₋₆-alkoxy;
- R³: signifies C₁₋₆-alkyl;
- R⁴: signifies hydrogen or C₁₋₆-alkyl;
- R⁵ and R⁶: each independently signify hydrogen or C₁₋₆-alkyl;
- r: has a value of from 0 to 250;
- s: has a value of from 0 to 20;
- r +S: has a value of at least 3;
- t: has a value of from 0 to 10;
- v: has a value of from 0 to10; and
- v+t: has a value of at least 3;
- n: has a value from 1 to 6;
with the proviso that in the case that s is 0 at least one X is A; in a topical composition comprising
a) 1 wt% to 30 wt% of the linear or cyclic polysiloxane compound of formula Ia or Ib, and
b) about 0.5 wt% to about 15 wt% of a tocopherol compound, for enhancing the penetration rate of the tocopherol compound.

As used herein, to tocopherol compound" refers to alpha tocopherol, mixed tocopherols, short-chain carboxylic acid ester of tocopherol such as e.g. tocopherol acetate, tocopherol propionate, and tocopherol butyrate. Preferred is tocopherol acetate.

As used herein, "C₁₋₆-alkyl" refers to groups such as methyl, ethyl, propyl, isopropyl, n-butyl, sec. butyl, tert. butyl, pentyl and neopentyl. The term "C₁₋₆-alkoxy" refers to the corresponding alkoxy groups.

The residue R signifies preferably C₁₋₄-alkyl, more preferably methyl.

The residues R¹ and R² can be the same or different. Preferably R¹ and R² have the same meaning and signify hydrogen, methoxy or ethoxy, more preferably hydrogen.

The residue R³ signifies preferably methyl or ethyl, more preferably ethyl.

The residue R⁴ signifies preferably hydrogen or methyl, more preferably hydrogen.

The residues R⁵ and R⁶ signify preferably hydrogen.

n has preferably a value of 1.

The polysiloxane compounds having a group A of the general formula IIa and IIb and their preparation are described in the European Patent EP 0538431 B1 and EP 0709080 AI. These polysiloxane compounds are preferred.

The polysiloxane compounds having a group A of the general formula IIc and their preparation are described in the European Patent EP 0358584 BL

In the linear polysiloxane compounds according to formula Ia the chromophore carrying residue A may be connected to the end groups of the polysiloxme (X=A, s=0) or may be statistically distributed (X=R, s>0). Linear polysiloxane compounds wherein the chromophore carrying residue A is statistically distributed are preferred. Said preferred polysiloxane compounds have at least one unit carrying the chromophore residue (s=1)*,* preferably s has a value of from about 2 to about 10, more preferably a statistical mean value of about 4. The number of r of the other silicone units present in the polysiloxane compounds is preferably about 5 to about 150, more preferably a statistical mean value of about 60.

The ratio of polysiloxane units having a chromophore residue A of the formula IIa to those having a chromophore residue A of the formula IIb is not critical. Said ratio may be about 1:1 to about 19:1, preferably about 2:1 to about 9:1, more preferably about 4:1.

The concentration of the polysiloxane compound in the topical composition is preferably about 2wt% to 20wt%, more preferably about 5 wt% to about 10 wt%.

The concentration of the tocopherol compound in the topical composition is preferably about 1wt% to about 10wt%, more preferably about 1wt% to about 5wt%.

The ratio of the tocopherol compound to the polysiloxane compound as defined above is not critical. For example the ratio is about 1:1 to about 1:20, preferably about 1:2 to about 1:3.

Thus, a preferred topical composition comprises about 1wt% to about 10wt% of a tocopherol compound and about 2wt% to 20wt% of a linear polysiloxane compound of the general formula Ia, wherein
- X: signifies methyl;
- A: signifies a group of the formula IIa or IIb;
- R: signifies C₁₋₄-alkyl;
- R¹ and R²: have the same meaning and signify hydrogen, methoxy or ethoxy;
- R³: signifies methyl or ethyl;
- R⁴: signifies hydrogen or methyl;
- R⁵ and R⁶: signify hydrogen;
- r: is about 5 to about 150;
- s: is about 2 to about 10;
- n: has a value of 1.

A more preferred topical composition comprises
about 1wt% to about 5wt% of tocopherol acetate; and
about 5wt% to about 10wt% of a linear polysiloxane compound of the general formula Ia, wherein
- X: signifies methyl;
- A: signifies a group of the formula IIa or IIb;
- R: signifies methyl;
- R¹ and R²: signify hydrogen;
- R³: signifies ethyl;
- R⁴: signifies hydrogen;
- R⁵ and R⁶: signify hydrogen;
- r: is a statistical mean value of about 4;
- s: is a statistical mean value of about 60;
- n: has a value of 1.

The polysiloxane compounds Ia or Ib wherein A is a residue of the formula IIa or IIb can be prepared as described in EP 0538431 B1 by silylation of the corresponding benzalmalonates. The following reaction scheme shows an example to build up a compound of the general formula la wherein R⁴, R⁵ and R⁶ signify hydrogen and n has a value of 1. 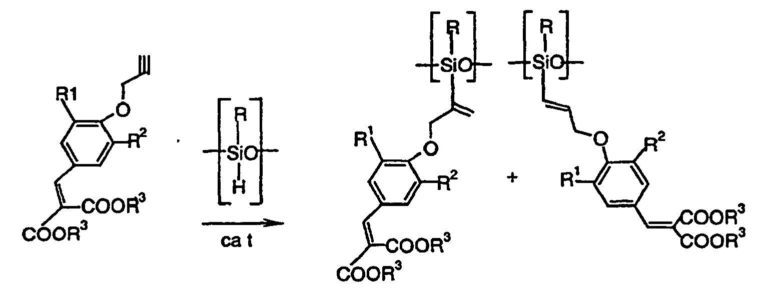 wherein R, R¹, R² and R³ are as defined above.

The silylation of the 4-(2-propynylox)phenyl methylene diethylester may be carried out employing known procedures for the addition of silicon bonded hydrogen atoms to groups containing aliphatic unsaturation. Such reactions are generally catalyzed by a platinum group metal or a complex of such a metal. Examples of catalysts which may be employed are platinum on carbon, chloroplatinic acid, platinum acetyl acetonate, complexes of platinum compounds with unsaturated compounds e.g. olefins and divinyl disiloxanes, complexes of rhodium and palladium compounds and complexes of platinum compounds supported on inorganic substrates. The addition reaction may be performed at reduced, atmospheric or increased pressure. A solvent can be used, e.g. toluene or xylene, in the reaction mixture although the presence of the solvent is not essential. It is also preferred to carry out the reaction at elevated reaction temperatures e.g. from about 50°C up to 150°C.

The production of the novel topical compositions comprises incorporating a polysiloxane compound as defined above and a tocopherol compound optionally in combination with other known UV-A and/or UV-B filters, in a cosmetic base which is usual for topical compositions such as e.g. for light screening agents. The preparation of said topical composition is well known to the suled artisan in this field.

Suitable UV-B filters, i.e. substances having absorption maxima between about 290 and 320 nm, are for example the following compounds:
--- Acrylates such as 2-ethylhexyl 2-cyano-3,3-diphenylacrylate (octocrylene, PARSOL 340), ethyl 2-cyano-3,3-diphenylacrylate and the like;
--- Camphor derivatives such as 4-methyl benzylidene camphor (PARSOL 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like;
--- Cinnamate derivatives such as octyl methoxycinnamate (PARSOL MCX), ethoxyethyl methoxycinnamate, diethanolamine methoxycinnamate (PARSOL Hydro), isoamyl methoxycinnamate and the like as well as cinnamic acid derivatives bond to siloxanes;
--- Pigments such as e.g. microparticulated TiO₂. The term "microparticulated" refers to a particle size from about 5 nm to about 200 nm, particularly from about 15 nm to about 100 nm. The TiO₂ particles may also be coated by metal oxides such as e.g. aluminum or zirconium oxides or by organic coatings such as e.g. polyols, methicone, aluminum stearate, alkyl silane. Such coatings are well known in the art.
--- Imidazole derivatives such as e.g. 2-phenyl benzimidazole sulfonic acid and its salts (PARSOL HS). Salts of 2-phenyl benzimidazole sulfonic acid are e.g. alkali salts such as sodium- or potassium salts, ammonium salts, morpholine salts, salts of primary, sec. and tert. amines like monoethanolamine salts, diethanolamine salts and the like.
--- Salicylate derivatives such as isopropylbenzyl salicylate, benzyl salicylate, butyl salicylate, octyl salicylate (NEO HFLIOPAN OS), isooctyl salicylate or homomenthyl salicylate (homosalate, HELIOPAN) and the like;
--- Triazone derivatives such as octyl triazone (UVINUL T-150), dioctyl butamido triazone (UVASORB HEB) and the like.

Suitable UV-A filters i.e. substances having absorption maxima between about 320 and 400 nm, are for example the following compounds.
---- Dibenzoylmethane derivatives such as 4-tert. butyl-4'-methoxydibenzoylmethane (PARSOL 1789), dimethoxydibenzoylmethane, isopropyldibenzoylinethane and the like;
---- Benzotriazole derivatives such as 2,2'-methylene-bis-(6-(2H-benzotriazole-2-yl)-4-(1,1,3,3,-tetramethylbutyl)-phenol (TINOSORB M) and the like;
--- Pigments such as e.g. microparticulated ZnO. The term "microparticulated" refers to a particle size from about 5 nm to about 200 nm, particularly from about 15 nm to about 100 nm. The ZnO particles may also be coated by metal oxides such as e.g. aluminum or zirconium oxides or by organic coatings such as e.g. polyols, methicone, aluminum stearate, alkyl silane. Such coatings are well known in the art.

As dibenzoylmethane derivatives are photolabile it is necessary to photostabilze these UV-A screening agents. Thus, the term "common UV-A screening agent" also refers to dibenzoylmethane derivatives such as e.g. Parsol 1789 stabilized by the following stabilizing agents,
--- 3,3-Diphenylacrylate derivatives (octocrylene) as described in the European Patent Publications EP 0514491 B1 and EP 0780119 Al;
---- Benzylidene camphor derivatives as described in US Patent No. US 5,605,680.

As cosmetic bases usual for light screening compositions in the scope of the present invention there can be used any conventional preparation which corresponds to the cosmetic requirements, e.g. creams, lotions, emulsions, salves, gels, solutions, sprays, sticks and milks; see also, Sunscreens, Development, Evaluation and Regulatory Aspects, ed. N.Y. Lowe, N.A. Shaath, Marcel Dekker, Inc. New York and Basel, 1990.

The following examples illustrate the invention in more detail, but do not limit its scope in any manner.

### Example 1

Preparation of an organosiloxane compound of the general formula Ia wherein R signifies methyl, s is 0, r is 20, X is A and A signifies a benzalmalonate residue of the formula IIa and IIb wherein R¹ and R² are hydrogen, R³ is ethyl and R⁴, R⁵ and R⁶ are hydrogen, n is 1 as described in EP 0538 431 B1.

5 g of {[4-(2-propynyloxy)phenyl] methylene}-diethyl ester were dissolved in 20 g of toluene and heated under nitrogen to about 80°C. 13.2 g of a hydrosiloxane having a degree of polymerization of 20 and 10 mpc (mole-%) SiH groups (3.62.% SiH) were then added dropwise after a platinum-divinyl-tetramethyl-disiloxane complex was also added, giving 10⁻⁴ mole of Pt per mole of SiH of the hydrosiloxane. The mixture was heated to reflux and maintained until all SiH had disappeared of the infrared spectroscopic analysis. It was then allowed to cool to room temperature. The toluene was then evaporated to leave after washing 16.5 g of a slightly brown polymer having the average structure A-[(CH₃)₂SiO]₂0-A, wherein A is a residue of the formula IIa1 and IIb1

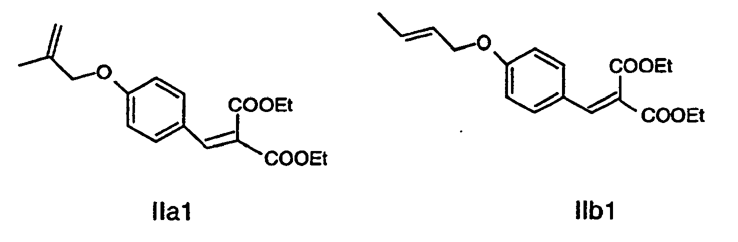

### Example 2

Preparation of an organosiloxane compound having the general formula Ia wherein R signifies methyl, r is 59, s is 4, X is methyl and A signifies a benzalmalonate residue of the formula IIa and IIb wherein R¹ and R² are hydrogen, R³ is ethyl, R⁴, R⁵ and R⁶ are hydrogen as described in the European publication EP 0709080 Al.

13.28 g of [4-(2-propynyloxy)phenyl]methylene}-diethyl ester were dissolved in 75 g of toluene and heated under nitrogen to about 70°C. 44 g of a hydrosiloxane having a degree of polymerization of 65 and 6 mpc SiH groups (2.36% SiH) were then added dropwise after a platinum-divinyl-tetramethyl-disiloxane complex was also added, giving 10⁻⁴ mole of Pt per mole of SiH of the hydrosiloxane. The mixture was heated to reflux and maintained until all SiH had disappeared of the infrared spectroscopic analysis. It was then allowed to cool to room temperature. The toluene was then evaporated to leave after washing 52 g of a brown, viscous polymer having the average structure (CH₃)₃SiO-[(CH₃)₂SiO]59-[(CH₃)ASiO]₄-Si(CH₃)₃, wherein A has the formula IIa1 and IIb1

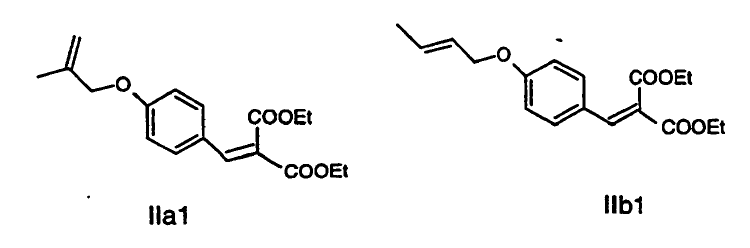

The ratio of compounds having a residue IIa1 to compounds having a residue IIb1 is about 4:1.

### Example 3

Topical composition according to the invention containing 5wt% polysiloxane.

A topical comprising 5wt% of a polysiloxane of formula Ia according to Example 2 and 2wt% of tocopherol acetate was prepared using the following ingredients:

| Part | %wt | Ingredient | INCI Name | Supplier |
|---|---|---|---|---|
| A | 2.5 | ARLACEL 60 | Sorbitan Stearate | ICI |
| | 17.5 | WITCONOL APM | PPG-3 Myristyl ether | WITCO |
| | 1.0 | Stearyl alkohol | Stearyl alkohol | |
| | 3.0 | Silicone oil | Dimethicone | DOW |
| | 2.0 | Tocopherol acetate | Tocopherol acetate | |
| | 5.0 | Compound of Ex. 2 | | |
| | | | | |
| B | 2.5 | TWEEN 60 | Polysorbate 60 | ICI |
| | 0.3 | KELTROL | Xanthan Gum | KELCO UK |
| | 64.2 | Water | | |
| | | | | |
| C | 2.0 | SEPIGEL 305 | Polyacrylamid & 13-14 isoparaffin+laureth-7 | SEPPIC |

### INCI: International Nomenclature of Cosmetic Ingredients

The compounds of Part A were added to and melted in a reactor. The compounds of Part B were mixed, heated to 85°C and added to Part A. Finally SEPIGEL 305 was added at a temperature of 50°C.

### Example 4

Topical composition according to the invention containing 10wt% polysiloxane.

A topical comprising 10wt% of a polysiloxane of formula Ia according to Example 2 and 2wt% of tocopherol acetate was prepared using the following ingredients:

| Part | %wt | Ingredient | INCI Name | Supplier |
|---|---|---|---|---|
| A | 2.5 | ARLACEL 60 | Sorbitan Stearate | ICI |
| | 17.5 | WITCONOLAPM | PPG-3 Myristyl ether | WITCO |
| | 1.0 | Stearyl alkohol | Stearyl alkohol | |
| | --- | Silicone oil | Dimethicone | DOW |
| | 2.0 | Tocopherol acetate | Tocopherol acetate | |
| | 10.0 | Compound of Ex. 2 | | |
| | | | | |
| B | 2.5 | TWEEN 60 | Polysorbate 60 | ICI |
| | 0.3 | KELTROL | Xanthan Gum | KELCO UK |
| | 62.2 | Water | | |
| | | | | |
| C | 2.0 | SEPIGEL 305 | Polyacrylamid & 13-14 isoparaffin+laureth-7 | SEPPIC |

The compounds of Part A were added to and melted in a reactor. The compounds of Part B were mixed, heated to 85°C and added to Part A. Finally SEPIGEL 305 was added at a temperature of 50°C.

### Example 5

Comparative Example

| Part | %wt | Ingredient | INCI Name | Supplier |
|---|---|---|---|---|
| A | 2.5 | ARLACEL 60 | Sorbitan Stearate | ICI |
| | 17.5 | WITCONOLAPM | PPG-3 Myristyl ether | WITCO |
| | 1.0 | Stearyl alkohol | Stearyl alkohol | |
| | 8.0 | Silicone oil | Dimethicone | DOW |
| | 2.0 | Tocopherol acetate | Tocopherol acetate | |
| | | | | |
| B | 2.5 | TWEEN 60 | Polysorbate 60 | ICI |
| | 0.3 | KELTROL | Xanthan Gum | KELCO UK |
| | 64.2 | Water | | |
| | | | | |
| C | 2.0 | SEPIGEL 305 | Polyacrylamid & 13-14 isoparaffin+laureth-7 | SEPPIC |

### Example 6

### Determination of the Penetration Rate

A lotion as described in Examples 3-5 was placed on freshly isolated physiologically intact hairless rat skin and the percutaneous absorption was assessed 16 h after application.

### Experimental conditions:

An area of 5 cm² was marked in the middle of the skin piece and the test material applied and distributed with a syringe. The syringe was weighed before and after each application of the test material to determine the exact amount of applied test product.

A small magnet was placed into the compartment of the bottom part of the penetration chamber. The skin specimen was then clamped onto the penetration chamber. Receptor-liquid was added to fill the compartment completely and assure full contact with the skin above. The receptor-phase was slightly moved by a magnetic stirrer throughout the whole experiment. The chamber was now connected to a thermostat and equilibrated at 32°C. After the penetration time the chamber was disconnected from the thermostat and the applied material recovered in the following way:
Skin surface: After the penetration chamber has been opened, the skin was taken off and fixed to a plate (cork) and wiped off with cotton wool several times, moistened with small amounts of EtOH/1420 1:1 to facilitate the cleaning of the surface. One last time the cotton wool is used dry. The cotton wool was extracted with 20 ml of acetontrile and the extract quantitatively analyzed by HPLC.
Stratum corneum: The horny layer of the skin was taken off with adhesive tape. A piece of tape was put on the skin with soft pressure and drawn off again. This procedure is called "stripping" and was repeated (10 ± 6 times) until the skin shines and a moderate afflux of moisture indicates the total removal of the horny layer. The adhesive tapes were extracted with 15 ml of acetontrile and quantitatively analyzed by HPLC.
Stripped skin: The application area was punched out, cut in small pieces with a scalpel and homogenized in 10 ml 100% acetontrile. It was then centrifuged and filtered, and the dear solution analyzed quantitatively by HPLC.
Receptor phase. The receptor-liquid (approx. 10 ml) is collected, the chamber rinsed with 3 x 1 ml acetontrile, which is added to the receptor-liquid and the volume is adjusted to 15ml. An aliquot of this solution is quantitatively analyzed by HPLC.

The tocopherol acetate content was analyzed by high performance liqtiid chromatography. The results (in % of total amount of tocopherol acetate found in the samples) are shown in the following table

| | Example 3 | Example 4 | Example 5 comparative |
|---|---|---|---|
| skin surface | 64.5 | 65.2 | 69.7 |
| Stratum Corneum | 9.5 | 7.4 | 9.5 |
| Stripped skin | 26.0 | 27.4 | 20.8 |
| Receptor Phase | 0 | 0 | 0 |

mean result of 4 measurements.

The above table clearly shows that the polysiloxane compound enhances the skin penetration of tocopherol acetate. The stripped skin contains 26-27% of tocopherol acetate applied compared to 20% of tocopherol acetate applied.

## Claims

1. Use of a linear or cyclic polysiloxane compound of the general formula Ia or Ib, 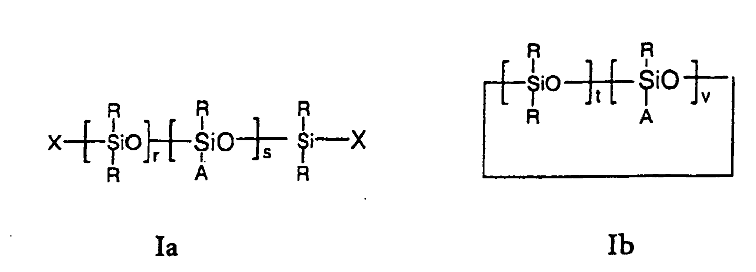
wherein
X signifies R or A;
A signifies a group of the general formula IIa, IIb or IIc; 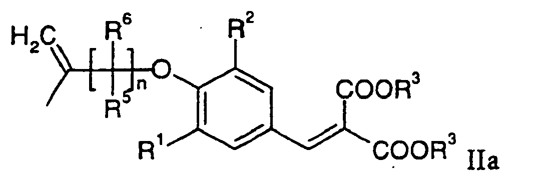 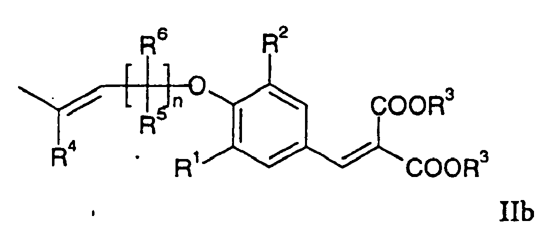 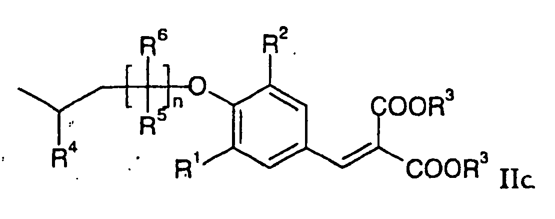
R signifies hydrogen, C₁₋₆ alkyl or phenyl;
R¹ and R² each independently signify hydrogen, hydroxy, C₁₋₆-alkyl or C₁₋₆ -alkoxy;
R³ signifies C₁₋₆-alkyl;
R⁴ signifies hydrogen or C₁₋₆-alkyl;
R⁵ and R⁶ each independently signify hydrogen or C₁₋₆-alkyl;
r has a value of from 0 to 250;
s has a value of from 0 to 20;
r +S has a value of at least 3;
t has a value of from 0 to 10;
v has a value of from 0 to 10; and
v+t has a value of at least 3;
n has a value from 1 to 6;
with the proviso that in the case that s is 0 at least one X is A;
in a topical composition comprising
a) 1 wt% to 30 wt% of the linear or cyclic polysiloxane compound of the general formula Ia or Ib and
b) 0.5 wt% to about 15 wt% of a tocopherol compound,
for enhancing the penetration rate of the tocopherol compound.

2. Use according to claim 1, wherein the tocopherol compound is alpha tocopherol, mixed tocopherols or a short-chain carboxylic acid ester of tocopherol.

3. Use according to claim 2, wherein the short-chain carboxylic acid ester of tocopherol is tocopherol acetate, tocopherol propionate or tocopherol butyrate, preferably tocopherol acetate.

4. Use according to any one of claims 1 to 3, wherein R signifies C₁₋₄-alkyl, preferably methyl.

5. Use according to any one of claims 1 to 4, wherein R¹ and R² have the same meaning and signify hydrogen, methoxy or ethoxy, preferably hydrogen.

6. Use according to any one of claims 1 to 5, wherein R³ signifies hydrogen or methyl, preferably hydrogen.

7. Use according to any one of claims 1 to 6, wherein R⁴ signifies hydrogen or methyl, preferably hydrogen.

8. Use according to any one of claims 1 to 7, wherein R⁵ and R⁶ signify hydrogen and n is 1.

9. Use according to any one of claims 1 to 8, wherein X signifies R, s has a value of from 1 to 20 and A is a residue of the formula IIa or IIb.

10. Use according to any one of claims 1 to 9, wherein the topical composition comprises
a) 2wt% to 20wt%, preferably 5 wt% to 10wt% of a linear or cyclic polysiloxane compound of the general formula Ia or Ib, and
b) 1 wt% to 10 wt%, preferably 1 wt% to 5 wt% of a tocopherol compound.

11. Use according to any one of claims 1 to 10, wherein the topical composition comprises 1 wt% to 10 wt% of a tocopherol compound and
2wt% to 20wt% of a linear polysiloxane compound of the general formula Ia, wherein
X signifies methyl;
A signifies a group of the formula IIa or IIb;
R signifies C₁₋₄-alkyl;
R¹ and R² have the same meaning and signify hydrogen, methoxy or ethoxy;
R³ signifies methyl or ethyl;
R⁴ signifies hydrogen or methyl;
R⁵ and R⁶ signify hydrogen;
r is 5 to 150;
s is 2 to 10;
n has a value of 1.

12. Use according to any one of claims 1 to 11, wherein the topical composition comprises 1 wt% to 5 wt% of tocopherol acetate; and
5wt% to 10wt% of a linear polysiloxane compound of the general formula Ia, wherein
X signifies methyl;
A signifies a group of the formula IIa or IIb;
R signifies C₁₋₄-alkyl;
R¹ and R² have the same meaning and signify hydrogen, methoxy or ethoxy;
R³ signifies methyl or ethyl;
R⁴ signifies hydrogen or methyl;
R⁵ and R⁶ signify hydrogen;
r is 5 to 150;
s is 2 to 10;
n has a value of 1.

13. Use according to any one of claims 1 to 12, wherein the topical composition comprises in addition common UV-A and/or UV-B filters.

## Patentansprüche

1. Verwendung einer linearen oder cyclischen Polysiloxanverbindung der allgemeinen Formel Ia oder Ib 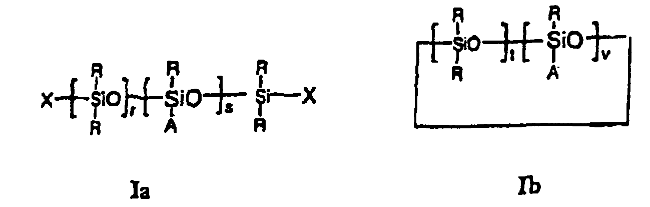
worin
X R oder A bedeutet;
A eine Gruppe der allgemeinen Formel IIa, IIb oder IIc bedeutet; 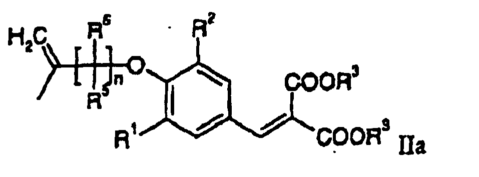 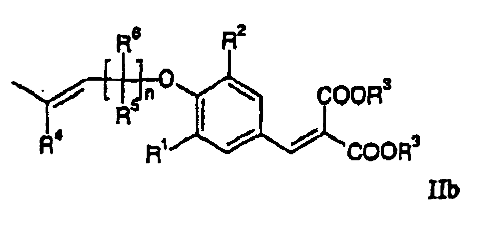 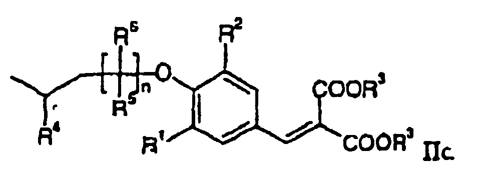
R Wasserstoff, C₁₋₆-Alkyl oder Phenyl bedeutet;
R¹ und R² jeweils unabhängig voneinander Wasserstoff, Hydroxy, C₁₋₆-Alkyl oder C₁₋₆₋Alkoxy bedeuten;
R³ C₁₋₆-Alkyl bedeutet;
R⁴ Wasserstoff oder C₁₋₆-Alkyl bedeutet;
R⁵ und R⁶ jeweils unabhängig voneinander Wasserstoff oder C₁₋₆-Alkyl bedeuten;
r einen Wert von 0 bis 250 aufweist;
s einen Wert von 0 bis 20 aufweist;
r + s einen Wert von mindestens 3 aufweist;
t einen Wert von 0 bis 10 aufweist;
v einen Wert von 0 bis 10 aufweist; und
v + t einen Wert von mindestens 3 aufweist;
n einen Wert von 1 bis 6 aufweist;
mit der Maßgabe, daß in dem Fall, daß s 0 ist, mindestens ein X A ist;
in einer topischen Zusammensetzung, umfassend
a) 1 Gew.% bis 30 Gew.-% der linearen oder cyclischen Polysiloxanverbindung der allgemeinen Formel Ia oder Ib und
b) 0,5 Gew.% bis etwa 15 Gew.% einer Tocopherolverbindung,
zur Verbesserung der Penetrationsrate der Tocopherolverbindung.

2. Verwendung nach Anspruch 1, wobei die Tocopherolverbindung alpha-Tocopherol, gemischte Tocopherole oder ein kurzkettiger Carbonsäureester von Tocopherol ist.

3. Verwendung nach Anspruch 2, wobei der kurzkettige Carbonsäureester von Tocopherol Tocopherolacetat, Tocopherolpropionat oder Tocopherolbutyrat, vorzugsweise Tocopherolacetat, ist.

4. Verwendung nach einem der Ansprüche 1 bis 3, wobei R C₁₋₄-Alkyl, vorzugsweise Methyl bedeutet.

5. Verwendung nach einem der Ansprüche 1 bis 4, wobei R¹ und R² dieselbe Bedeutung aufweisen und Wasserstoff, Methoxy oder Ethoxy, vorzugsweise Wasserstoff, bedeuten.

6. Verwendung nach einem der Ansprüche 1 bis 5, wobei R³ Wasserstoff oder Methyl, vorzugsweise Wasserstoff bedeutet.

7. Verwendung nach einem der Ansprüche 1 bis 6, wobei R⁴ Wasserstoff oder Methyl, vorzugsweise Wasserstoff bedeutet.

8. Verwendung nach einem der Ansprüche 1 bis 7, wobei R⁵ und R⁶ Wasserstoff bedeuten und n 1 ist.

9. Verwendung nach einem der Ansprüche 1 bis 8, wobei X R bedeutet, s einen Wert von 1 bis 20 aufweist und A ein Rest der Formel IIa oder IIb ist.

10. Verwendung nach einem der Ansprüche 1 bis 9, wobei die topische Zusammensetzung
a) 2 Gew.-% bis 20 Gew.-%, vorzugsweise 5 Gew.-% bis 10 Gew.% einer linearen oder cyclischen Polysiloxanverbindung der allgemeinen Formel Ia oder Ib, und
b) 1 Gew.-% bis 10 Gew.%, vorzugsweise 1 Gew.% bis 5 Gew.% einer Tocopherolverbindung
umfaßt.

11. Verwendung nach einem der Ansprüche 1 bis 10, wobei die topische Zusammensetzung 1 Gew.% bis 10 Gew.-% einer Tocopherolverbindung und 2 Gew.-% bis 20 Gew.-% einer linearen Polysiloxanverbindung der allgemeinen Formel Ia umfaßt, worin
X Methyl bedeutet;
A eine Gruppe der Formel IIa oder IIb bedeutet;
R C₁₋₄-Alkyl bedeutet;
R¹ und R² dieselbe Bedeutung aufweisen und Wasserstoff, Methoxy oder Ethoxy bedeuten;
R³ Methyl oder Ethyl bedeutet;
R⁴ Wasserstoff oder Methyl bedeutet;
R⁵ und R⁶ Wasserstoff bedeuten;
r 5 bis 150 ist;
s 2 bis 10 ist;
n einen Wert von 1 aufweist.

12. Verwendung nach einem der Ansprüche 1 bis 11, wobei die topische Zusammensetzung 1 Gew.% bis 5 Gew.-% Tocopherolacetat und 5 Gew.-% bis 10 Gew.-% einer linearen Polysiloxanverbindung der allgemeinen Formel la umfaßt, worin
X Methyl bedeutet;
A eine Gruppe der Formel IIa oder IIb bedeutet;
R C₁₋₄-Alkyl bedeutet;
R¹ und R² dieselbe Bedeutung aufweisen und Wasserstoff, Methoxy oder Ethoxy bedeuten;
R³ Methyl oder Ethyl bedeutet;
R⁴ Wasserstoff oder Methyl bedeutet;
R⁵ und R⁶ Wasserstoff bedeuten;
r 5 bis 150 ist;
s 2 bis 10 ist;
n einen Wert von 1 aufweist.

13. Verwendung nach einem der Ansprüche 1 bis 12, wobei die topische Zusammensetzung außerdem bekannte UV-A- und/oder UV-B-Filter umfaßt.

## Revendications

1. Utilisation d'un composé polysiloxane linéaire ou cyclique de formule générale Ia ou Ib 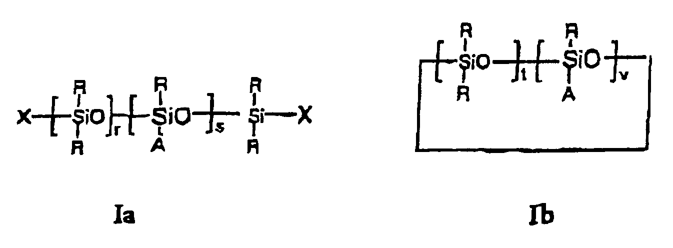
dans lesquelles :
X signifie R ou A ;
A signifie un groupe de formule générale IIa, IIb ou IIc 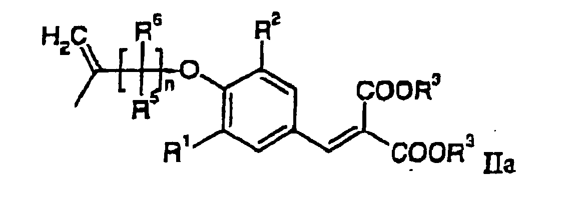 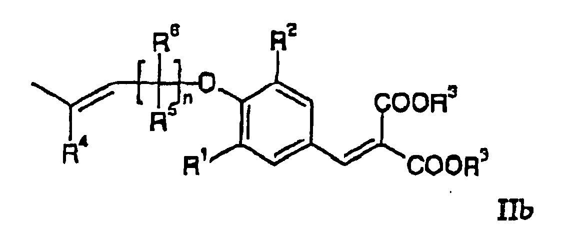 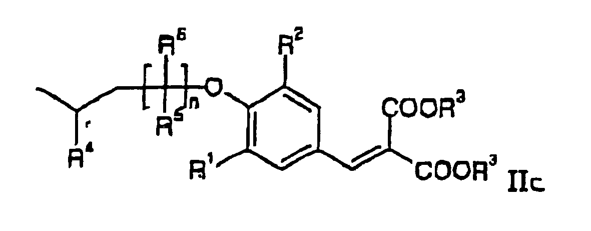
R signifie un groupe hydrogène, phényle ou alkyle en C₁₋₆;
R¹ et R² signifient chacun indépendamment un groupe hydrogène, hydroxy, alkyle en C₁₋₆ ou alkoxy en C₁₋₆;
R³ signifie un groupe alkyle en C₁₋₆ ;
R⁴ signifie un groupe hydrogène ou alkyle en C₁₋₆;
R⁵ et R⁶ signifient chacun indépendamment un hydrogène ou un alkyle en C₁₋₆
r a une valeur de 0 à 250 ;
s a une valeur de 0 à 20 ;
r + s a une valeur d'au moins 3 ;
t a une valeur de 0 à 10 ;
v a une valeur de 0 à 10 ; et
v +t a une valeur d'au moins 3 ;
n a une valeur de 1 à 6 ;
à la condition que dans les cas dans lesquels s est 0, au moins un X est A;
dans une composition topique comprenant :
a) de 1 % en poids à 30 % en poids du composé polysiloxane linéaire ou cyclique de formule générale Ia ou Ib ; et
b) 0,5 % en poids à environ 15 % en poids d'un composé tocophérol,
pour augmenter le rythme de pénétration du composé tocophérol.

2. Utilisation selon la revendication 1, dans laquelle le composé tocophérol est un alpha-tocophérol, un mélange de tocophérols ou un ester d'acide carboxylique à chaîne courte de tocophérol.

3. Utilisation selon la revendication 2, dans laquelle l'ester d'acide carboxylique à chaîne courte de tocophérol est de l'acétate de tocophérol, du propionate de tocophérol ou du butyrate de tocophérol, de préférence de l'acétate de tocophérol.

4. Utilisation selon l'une quelconque des revendications 1 à 3, dans laquelle R signifie un alkyle en C₁₋₄, de préférence un méthyle.

5. Utilisation selon l'une quelconque des revendications 1 à 4, dans laquelle R¹ et R² ont la même signification et signifient un groupe hydrogène, éthoxy ou méthoxy, de préférence hydrogène.

6. Utilisation selon l'une quelconque des revendications 1 à 5, dans laquelle R³ signifie un groupe hydrogène ou méthyle, de préférence hydrogène.

7. Utilisation selon l'une quelconque des revendications 1 à 6, dans laquelle R⁴ signifie un groupe hydrogène ou méthyle, de préférence hydrogène.

8. Utilisation selon l'une quelconque des revendications 1 à 7, dans laquelle R⁵ et R⁶ signifient un groupe hydrogène et n est 1.

9. Utilisation selon l'une quelconque des revendications 1 à 8, dans laquelle X signifie R, s a une valeur de 1 à 20 et A est un résidu de la formule IIa ou IIb.

10. Utilisation selon l'une quelconque des revendications 1 à 9, dans laquelle la composition topique comprend :
a) de 2 % en poids à 20 % en poids, de préférence de 5 % en poids à 10 % en poids d'un composé polysiloxane linéaire ou cyclique ayant la formule générale Ia ou Ib, et
b) de 1 % en poids à 10 % en poids, de préférence de 1 % en poids à 5 % en poids d'un composé tocophérol.

11. Utilisation selon l'une quelconque des revendications 1 à 10, dans laquelle la composition typique comprend de 1 % en poids à 10 % en poids d'un composé tocophérol, et
de 2 % en poids à 20 % en poids d'un composé polysiloxane linéaire ou cyclique de formule générale Ia, dans laquelle :
X signifie un groupe méthyle
A signifie un groupe de formule IIa ou IIb
R signifie un groupe alkyle en C₁₋₄
R' et R² ont la même signification et signifient un groupe hydrogène, méthoxy ou éthoxy ;
R³ signifie un groupe méthyle ou éthyle ;
R⁴ signifie un groupe hydrogène ou méthyle ;
R⁵ et R⁶ signifient un groupe hydrogène ;
r va de 5 à 150 ;
s va de 2 à 10
n a une valeur de 1.

12. Utilisation selon l'une quelconque des revendications 1 à 10, dans laquelle la composition topique comprend de 1 % en poids à 5 % en poids d'acétate de tocophérol ; et
de 5 % en poids à 10 % en poids d'un composé polysiloxane linéaire ou cyclique de formule générale Ia, dans laquelle :
X signifie un groupe méthyle ;
A signifie un groupe de formule IIa ou IIb ;
R signifie un groupe alkyle en C₁₋₄ ;
R¹ et R² ont la même signification et signifient un groupe hydrogène, méthoxy ou éthoxy ;
R³ signifie un groupe méthyle ou éthyle ;
R⁴ signifie un groupe hydrogène ou méthyle ;
R⁵ et R⁶ signifient un groupe hydrogène ;
r va de 5 à 150;
s va de 2 à 10 ;
n a une valeur de 1.

13. Utilisation selon l'une quelconque des revendications 1 à 12, dans laquelle la composition topique comprend en plus des filtres contre les UV-A et/ou les UV-B courants.
